# EUROPEAN PATENT SPECIFICATION

(11) **EP 4 272 798 B1**
(45) Date of publication and mention of the grant of the patent: **26.11.2025**
(21) Application number: 22748818.6
(22) Date of filing: 11.01.2022
(51) Int. Cl.: A61M 25/00

(54) **CATHETER WITH A REINFORCEMENT LAYER**
KATHETER MIT VERSTÄRKUNGSSCHICHT
CATHÉTER AVEC UNE COUCHE DE RENFORCEMENT

(30) Priority: 05.02.2021 CN 202110164311
(43) Date of publication of application: 08.11.2023
(73) Proprietor: MicroPort NeuroTech (Shanghai) Co., Ltd., Shanghai 201318 (CN)
(72) Inventor: LIN, Heng, Shanghai 201318 (CN); LIU, Yunyun, Shanghai 201318 (CN); WANG, Yiqun Bruce, Shanghai 201318 (CN); LUO, Xueli, Shanghai 201318 (CN)
(74) Representative: Marks & Clerk LLP
(86) International application number: PCT/CN2022/071231
(87) International publication number: WO 2022/166539

(56) References cited:
- WO-A1-00/43061
- CN-A- 103 706 017
- CN-A- 104 548 315
- CN-A- 104 740 748
- CN-A- 108 514 677
- CN-A- 109 173 003
- CN-U- 204 951 906
- CN-U- 215 083 905
- JP-A- 2010 042 115
- US-A1- 2008 251 966
- US-A1- 2016 279 317
- US-A1- 2020 206 002

## Description

### TECHNICAL FIELD

The present invention relates to the field of medical instruments and, in particular, to a catheter reinforcement layer and a catheter including the catheter reinforcement layer.

### BACKGROUND

With the support of Digital Subtraction Angiography (DSA) system, minimally invasive interventional surgery is to deliver implanted medical devices or therapeutic drugs to the lesion with minimal trauma through the intravascular catheter delivery system, so that the lesion is treated mechanically or chemically. As an important part of the delivery system, catheters have been widely used in various minimally invasive interventional treatments. At present, such interventional procedures usually form the entrance by puncturing small blood vessels (such as femoral artery and radial artery). After passing through the entrance, the catheter is delivered along the blood vessel to the target lesion with the assistance of the sheath and guide wire. The catheter then acts as an access for other devices such as stents, coils, other catheters, etc.

US2020/206002A1 describes self-sealing tubular grafts, patches, and methods for making and using them. CN109173003A describes an intermediate catheter capable of catheter passage through distal intracranial vessels or delivery of other instruments to distal vessels. US2016279317A1 describes a system for providing a continuous flow of blood between two locations in a cardiovascular system of a patient. WO0043061A1 describes an intravascular catheter with composite reinforcement.

At present, in order to meet the requirement of smooth delivery of catheters to the lesion during clinical use, existing medical catheters are usually designed with a more rigid proximal end and a more flexible distal end, for example, the stiffness gradually decreases from the proximal end to the distal end. A catheter consists of multiple segments with different stiffness. In order to allow the distal and proximal ends of the catheter to be different in flexibility, usually the outer layer and/or inner layer of the catheter will be formed of materials with different stiffness (in the axial direction). Moreover, the distal end of the catheter needs to be as flexible as possible, and the overall catheter needs to have a good transition of flexibility. However, due to the material and design characteristics, the axial modulus of flexible catheters is low, and the joints of catheter segments (e.g., joints between segments of the inner or outer layer or transitions of a certain characteristic) and the thinner part of the catheter body tend to become a weak point for stress, so that in clinical operations, the catheter is prone to axial deformation under the action of axial force, or even torn off, which has a serious adverse effect on clinical operations.

### SUMMARY OF THE INVENTION

An object of the present invention is to provide a catheter reinforcement layer and a catheter to solve the problem of insufficient axial tensile performance or even breakage of the existing catheter.

In order to solve the above-mentioned technical problem, the present invention provides a catheter as defined in claim 1. For better understanding of the invention, the present disclosure provides also further examples of a catheter, which comprises a catheter reinforcement layer, comprising a spring component and a plurality of axial components, wherein each of the plurality of axial components extends along the spring component from a proximal end to a distal end, and has at least one intersection with 69581595-1 the spring component.

Optionally, a material of each of the axial components is metal and/or polymer filament.

Optionally, each of the axial components has a filamentous structure shaped as any one or a combination of a straight line, wave line or spiral line.

Optionally, each of the axial components is arranged parallel to an axial direction of the spring component; or
each of the axial components is arranged at an angle of 0-45° with an axial direction of the spring component; or
at least a part of each of the axial components is arranged at an angle of 0-45° with an axial direction of the spring component, and other parts of the plurality of axial components are parallel to the axial direction of the spring component.

Optionally, the axial member has 1-80,000 said intersections with the spring component.

Optionally, each of the axial components, from the proximal end to the distal end, is shaped as a straight line or spiral line attached to an inner surface or an outer surface of the spring component; or
each of the axial components, from the proximal end to the distal end, is shaped as a wave line attached between an inner surface and an outer surface of the spring component in a staggered manner; or
each of the axial components, from the proximal end to the distal end, is arranged in a clearance of the spring component, and the plurality of axial components and the spring component are in a same plane.

Optionally, the plurality of axial components are arranged symmetrically or asymmetrically along a circumference of the spring component; and/or
the plurality of axial components are sequentially arranged at intervals along an axial direction of the spring component.

Optionally, the plurality of axial components are arranged symmetrically or asymmetrically along a circumference of the spring component at the predetermined position; and/or
the plurality of axial components are sequentially arranged at intervals along an axial direction of the spring component at the predetermined position.

Optionally, the plurality of axial components are arranged spirally along an axial direction of the spring component.

Optionally, axial distances between the plurality of axial components are the same or different, and/or
circumferential angular distances between the plurality of axial components are the same or different.

Optionally, the axial distances between the axial components located at the proximal end is smaller than the axial distances of the axial components located at the distal end; and/or
the circumferential angular distances between the axial components located at the proximal end is smaller than the axial distances of the axial components located at the distal end.

Optionally, a number of the axial components ranges from 2 to 16,000.

Optionally, an axial distance between two axial components adjacent in an axial direction of the spring component is in a range of 0.00254 - 0.254 cm (0.001-0.1 inches).

Optionally, a material of at least one of the at least one axial member is a radioautographic material.

Optionally, each of the axial components is a monofilament or a twisted filament composed of a plurality of monofilaments.

Optionally, a diameter of the monofilament is in a range of 0.00127 - 0.00762 cm (0.0005-0.003 inches), and a number of the monofilaments contained in the twisted filament is in a range of 1-20.

Optionally, each of the axial components is connected to the spring component by means of gluing, polymerization, bonding or laser welding, or is integrally formed with the spring component by means of cutting.

The catheter according to the present invention comprises an inner layer, a reinforcement layer and an outer layer that are sequentially arranged from inside to outside and are all in a shape of a tube, and wherein the reinforcement layer comprises the above catheter reinforcement layer.

Optionally, the axial component comprises a control wire extended reversely from a side close to a catheter operator, so that the catheter operator can adjust a rotation direction of the catheter through the control wire.

Optionally, the inner layer is a polymer material layer having a thickness of 0.000254 - 0.00508 cm (0.0001-0.002 inches).

Optionally, the inner layer has a thickness of 0.000762 - 0.001524 cm (0.0003-0.0006 inches).

Compared to the prior art, the present invention offers at least one of the following advantages:
1. The catheter reinforcement layer and the catheter according to the present invention have a novel reinforcement layer, which increases the axial modulus of the catheter by introducing one or more axial components into the reinforcement layer of the spring component of the existing catheter. It avoids the axial deformation or even breakage of the catheter caused from the axial force of the tubular body of the catheter.
2. One or more axial components may be arranged on the mechanically weak point of the catheter, it is thus possible to prevent stress concentration on the catheter, thereby preventing the breakage of the catheter during the delivery or withdrawal of a device.
3. Introducing one or more axial components extending in the axial direction into the reinforcement layer of the catheter can improve the efficiency in transmission of the axial force of the catheter and optimize the transmission performance of the catheter.
4. When the thickness of the inner layer of the catheter is particularly small, one or more axial components extending in the axial direction are introduced into the reinforcement layer of the catheter, which can not only ensure the flexibility of the catheter, but also prevent the axial deformation and breakage of the catheter.

### BRIEF DESCRIPTION OF THE DRAWINGS

Fig. 1 is a structural schematic diagram of a catheter reinforcement layer and a catheter using the catheter reinforcement layer according to a reference embodiment;
Figs. 2a-2c are structural schematic diagrams of the catheter reinforcement layer according to another reference embodiment;
Fig. 3 is structural schematic diagrams of a catheter reinforcement layer according to another reference embodiment;
Fig. 4 is a structural schematic diagram of a catheter reinforcement layer according to an embodiment of the present invention;
Fig. 5 is a structural schematic diagram of a catheter reinforcement layer according to another embodiment of the present invention;
Fig. 6 is a structural schematic diagram of a catheter reinforcement layer according to another embodiment of the present invention;
Fig. 7 is a structural schematic view of a catheter according to an embodiment of the present invention.

List of reference numerals:
10, catheter; 100, catheter reinforcement layer;
200, outer layer; 110, spring component;
120, axial component; 130, control wire.

### DETAILED DESCRIPTION

The catheter reinforcement layer and the catheter including the catheter reinforcement layer of the present disclosure will be further described in detail below. The disclosure will now be described in more detail with reference to the accompanying drawings, in which preferred embodiments of the invention are shown. It should be understood that those skilled in the art may modify the invention described herein and still achieve the beneficial effects of the invention. Therefore, the following description should be understood as the broad knowledge of those skilled in the art, but not as a limitation of the present invention.

In the interest of clarity, not all features of a practical implementation are described. In the following description, well-known functions are not described in detail since they would obscure the invention in unnecessary detail. It should be appreciated that in the development of any practical embodiment, numerous implementation details must be worked out to achieve the developer's specific goals, such as changing from one embodiment to another in accordance with system-related or business-related constraints. Additionally, it should be recognized that such a development effort might be complex and time consuming, but would nevertheless be merely a routine undertaking for those skilled in the art.

As used herein and in the appended claims, the singular forms of "a", "an", and "the", include plural references unless the context clearly dictates otherwise. As used herein and in the appended claims, the term "or" refers to "and/or" unless the context clearly dictates otherwise. The term "plurality" is usually used to include two or more objects, unless the content clearly states otherwise. The term "several" is generally used to include an indefinite number of objects, unless the content clearly states otherwise. The term "proximal end" generally refers to the end close to the operator of the medical device, and "distal end" generally refers to the end of the device that first enters the human body, unless the content clearly indicates otherwise.

As mentioned in the background, at present, in order to meet the requirement that the catheter can be smoothly delivered to the lesion in clinical use, existing medical catheters are usually designed with a more rigid proximal end and a more flexible distal end, for example, the stiffness gradually decreases from the proximal end to the distal end. A catheter consists of multiple segments with different stiffness. In order to allow the distal and proximal ends of the catheter to be different in flexibility, usually the outer layer and/or inner layer of the catheter will be formed of materials with different stiffness (in the axial direction). Moreover, the distal end of the catheter needs to be as flexible as possible, and the overall catheter needs to have a good transition of flexibility. However, due to the material and design characteristics, the axial modulus of flexible catheters is low, and the joints of catheter segments (e.g., joints between segments of the inner or outer layer or transitions of a certain characteristic) and the thinner part of the catheter body tend to become a weak point for stress, so that in clinical operations, the catheter is prone to axial deformation under the action of axial force, or even torn off, which has a serious adverse effect on clinical operations.

In view of the above problems, the researchers of the present invention found that the overall mechanical properties of the catheter are related to the modulus and stiffness of the polymer material, and the strength of the metal wire of the reinforcement layer and the metal coverage. In the prior art, the catheter is mainly composed of an inner layer, a reinforcement layer and an outer layer, which form a triple-layer structure. For catheters with a reinforcement layer in the form of a spring, due to its special structure, it can meet the requirements of high flexibility catheters, but the reinforcement layer in the form of a spring contributes little to the overall resistance of the catheter to axial deformation. The axial tensile resistance of the catheter with the reinforcement layer in the form of a spring mainly depends on the strength of the polymer material of the inner layer and the outer layer. When the inner and outer layers are made of polymer materials with low stiffness, or when there is an axial transition structure in the inner and outer layers, the axial tensile performance of the catheter is insufficient, and in severe cases, a failure mode of breakage will occur.

Therefore, the present invention provides a catheter reinforcement layer and a catheter including the catheter reinforcement layer, so as to solve the problem of insufficient axial tensile performance of the existing catheters, and in severe cases, the failure mode of breakage will occur.

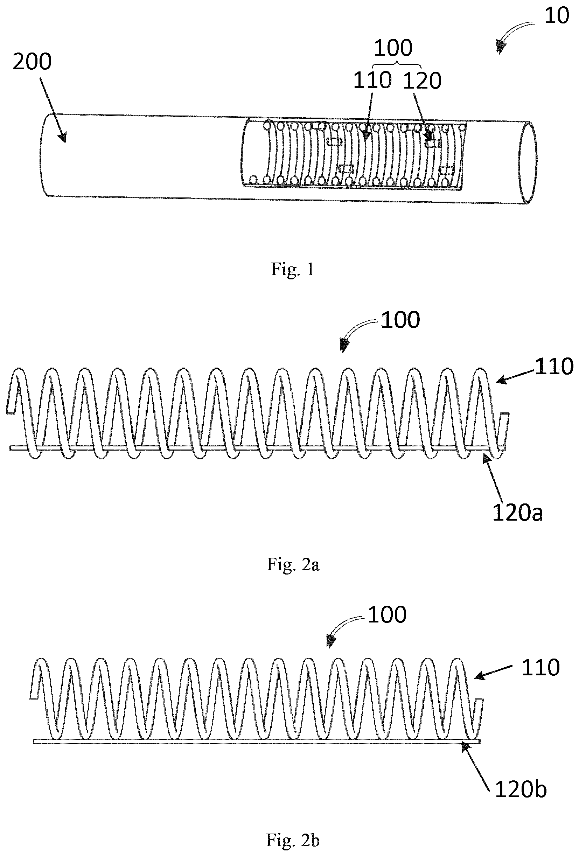

Referring to Fig. 1, which is a structural schematic diagram of a catheter reinforcement layer and a catheter using the catheter reinforcement layer according to an embodiment of the present invention. As shown in Fig. 1, the present disclosure provides a catheter, which can be used as a delivery catheter or for other medical purposes. The catheter is, for example, a hollow tube. In this embodiment, the catheter is, for example, a delivery catheter, which is used to provide a delivery path for an intravascular interventional device, and for release and withdrawal of the intravascular interventional device, and the like. The catheter includes, but is not limited to, a diameter of 6F-12F, so that the catheter can allow interventional instruments to be delivered into blood vessels in neurological interventional surgery, cardiac interventional surgery, aortic interventional surgery or peripheral vascular interventional surgery. In other embodiments, the diameter of the catheter can be modified according to actual needs.

As shown in Fig. 1, the present disclosure provides a catheter reinforcement layer and a catheter using the catheter reinforcement layer. The catheter includes an inner layer, a reinforcement layer and an outer layer which are sequentially arranged from the inside to the outside and are all in the shape of a tube. The catheter reinforcement layer includes a catheter reinforcement layer that includes a spring component and a plurality of axial components. Optionally, each of the plurality of axial components extends from the proximal end to the distal end along the spring component, and has at least one intersection with the spring component. Specifically, the axial components in the axial direction may be arranged parallel to the axial direction of the catheter, or may be arranged at a certain angle in a range of 0-45° such as 3°, 5°, 10°, 15°, 30°, 45° with the axial direction of the catheter. Optionally, at least a part of the plurality of axial components may form a certain angle in a range of 0-45° with the axial direction of the spring component, and other parts of the plurality of axial components are arranged along the axial direction parallel to the spring component. The axial components may have a filamentous structure, and be shaped as a straight line, wavy line or spiral line or a combination thereof. In an exemplary embodiment, the spring component may be shaped as a spiral line in a clockwise manner arranged between the inner layer and the outer layer of the catheter, or may be shaped as other curved lines such as a spiral line in a counterclockwise manner between the inner layer and the outer layer of the catheter. The spring component may be arranged uniformly or non-uniformly along the axial direction of the catheter, which is not specifically limited in the present invention.

Specifically, the material of the inner layer of the catheter may be any one or any combination of polymer materials such as polytetrafluoroethylene, polyurethane, polyamide, polyolefin, polyolefin elastomer and thermoplastic elastomer, and the thickness of the inner layer may range from 0.000254 - 0.00508 cm (0.0001 inches to 0.002 inches). Optionally, the thickness of the inner layer may range from 0.000762 - 0.001524 cm (0.0003 inches to 0.0006 inches). The material of the outer layer of the catheter may be any one or any combination of polymer materials such as polyurethane, polyolefin, polyolefin elastomer, thermoplastic elastomer and polyamide. In an exemplary embodiment, the material of the inner layer and the material of the outer layer may be preferably thermoplastic elastomers. The material of the spring component of the reinforcement layer may be metal, for example, any one of stainless steel, nickel-titanium alloy, cobalt-chromium alloy and tungsten. Optionally, the material of the spring component may be polymer filaments, for example, made from polyethylene, polyamide, liquid crystalline polymer. Optionally, the material of the spring component may be a combination of metal and polymer filaments. Moreover, the material of the axial components of the reinforcement layer may be metal and/or polymer filaments. Specifically, the material of the axial components may be any one of metals such as stainless steel, nickel-titanium alloy, cobalt-chromium alloy, tungsten, silver and gold, or any one of polymer filaments such as polyethylene, polyethylene, liquid crystals. Optionally, the material of the axial components may be a combination of the metal and the polymer filaments. The material of some of the axial components may be metal, and the material of some of the axial components may be polymer filaments. The material of the axial components may be a radioautographic material.

Optionally, there may be 1-80000, for example 1, 4, 10, 100, 200, 500, 1000, 2000, 5000, 10000, 20000, 40000, 60000, 80000 intersections between the axial components and the spring component.

It should be noted that the catheter according to the present invention is soft because the materials of the inner and outer layers are same as those of the existing catheter, while the axial components are provided on the (catheter) reinforcement layer in the axial direction of the spring component, thereby avoiding the axial deformation, or even the breakage of the catheter caused from the axial force of the tubular body of the catheter. Therefore, as long as the axial components are provided in any way in the axial direction of the spring component, the object of the invention of the present invention can be achieved.

Since the main improvement of the catheter according to the present invention lies in the (catheter) reinforcement layer among the triple-layer structure, the following will focus on the structure of the catheter reinforcement layer, and take the spring component shaped as a spiral line in a clockwise manner as an example to describe the various combinations of spring component and axial components in the reinforcement layer of the catheter according to the present invention in detail with reference to the accompanying drawings.

### Reference Embodiment 1

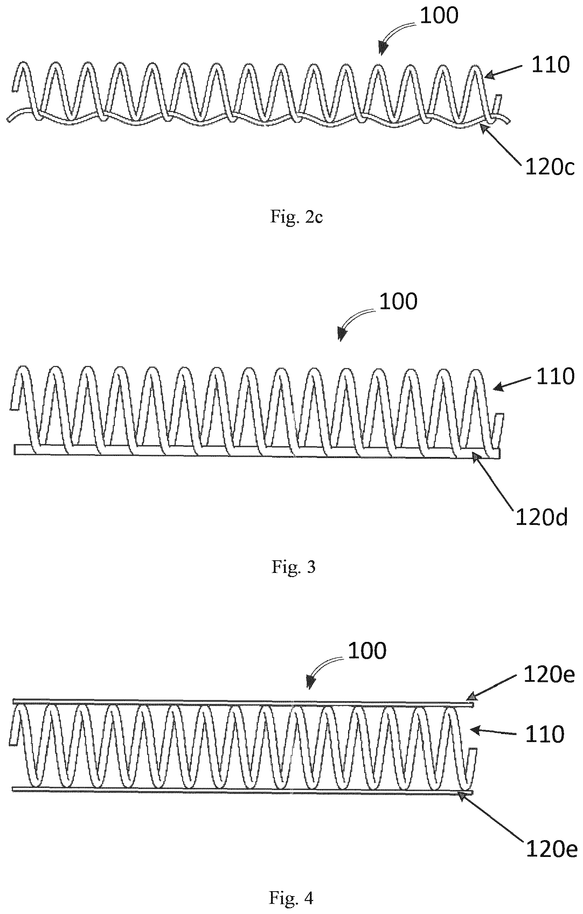

Referring to Figs. 2a-2c, which are structural schematic diagrams of a catheter reinforcement layer according to an embodiment of the present disclosure. As shown in Figs. 2a-2c as well as Fig. 1, the catheter reinforcement layer 100 includes a spring component 110 and an axial component 120 extending from the proximal end to the distal end along the spring component 110 and extending across the spring component 110. Specifically, as shown in Fig. 2a, the axial component 120a, from the proximal end to the distal end along the spring component 110, may be shaped as a straight line or spiral line attached between (the inner surface of) the spring component 110 and the inner layer (not shown). Alternatively, as shown in Fig. 2b, the axial component 120b, from the proximal end to the distal end, may be shaped as a straight line or spiral line attached between (the outer surface of) the spring component 110 and the outer layer (not shown). Alternatively, as shown in Fig. 2c, the axial component 120c, from the proximal end to the distal end, may be shaped as a wave line attached between the inner surface and the outer surface of the spring component 110 in a staggered manner.

### Reference Embodiment 2

Referring to Fig. 3, which is a structural schematic diagram of a catheter reinforcement layer according to an embodiment of the present disclosure. As shown in Fig. 3, the catheter reinforcement layer 100 is a combination of the spring component 110 and the axial component 120d. The axial component 120d may be arranged between the clearances of the spring component 100 from the proximal end to the distal end along the spring component 110, and the axial component 120d is in the same plane with the spring component 110. Compared with the catheter reinforcement layer 100 shown in Figs. 2a-2c, the catheter reinforcement layer 110 shown in Fig. 3 has an advantage in that the outer diameter of the catheter including the catheter reinforcement layer is not increased although the axial component 120 is included.

It should be noted that, in addition to the case where the axial component 120 extends fully across the spring component 110 from the proximal end to the distal end to form the catheter reinforcement layer 100 together with the spring component 110 in the manner described in Embodiment 1 and Embodiment 2 above, the axial component 120 may alternatively extend partially across the spring component 110 to form the catheter reinforcement layer 100 together with the spring component 110. The disclosure. is not specifically limited hereto.

Preferably, the number of the axial components may be in a range of 2-16000, such as 2, 4, 8, 20, 100, 800, 2000, 5000, 10000, 12000, 16000. The plurality of axial components 120 may be arranged symmetrically or asymmetrically along the circumference of the spring component 110, and/or the axial components 120 may be arranged at intervals along the axial direction of the spring component 110. It should be emphasized that the "interval" mentioned in this specification means that the proximal ends of two adjacent axial components are not at the same axial position, but arranged axially in a staggered manner. In some embodiments, two adjacent axial components may have no "overlap" in the axial direction. In other embodiments, two adjacent axial components may have partial "overlap" in the axial direction. The "overlap" here means that, at a same axial position, cross-sections of the above-mentioned two adjacent axial components are both located in the cross-section of the reinforcement layer.

The following provides examples where a plurality of axial components 120 are arranged symmetrically or asymmetrically along the circumference of the spring component 110 to introduce the positional relation between the axial components and the spring component 110. For details, see the following embodiments.

### Embodiment 3

Fig. 4 is a structural schematic diagram of a catheter reinforcement layer according to an embodiment of the present disclosure. As shown in Fig. 4, preferably, the catheter reinforcement layer 100 includes a spring component 110 and a plurality of axial components 120e that extends across the spring component 110 from the proximal end to the distal end and are arranged symmetrically. Since the axial components 120e are disposed symmetrically on the surface of the spring component 100, the structural stability of the catheter including the catheter reinforcement layer can be ensured. Similar to Reference Embodiment 1 and Reference Embodiment 2, it avoids the axial deformation of the catheter caused from the axial shear stress of the tubular body of the catheter when a device is delivered in the tubular body of the catheter, which provides the catheter with an increased resistance to axial tension. Therefore, the elongation of the catheter is reduced, which avoids the risk of fatigue failure of the catheter caused from axial stretch by the compatible device.

Optionally, the number of axial components 120e may range from 2 to 10, for example, 2, 3, 5, 6, 8, or 10.

It can be understood that, the plurality of axial components 120 can be arranged symmetrically or asymmetrically along the circumferential surface of the spring component 110 at any position. However, in the prior art, the catheter is usually designed to have a more rigid proximal end and a more flexible distal end. In other words, the hardness gradually decreases from the proximal end to the distal end. In this case, the catheter is composed of a plurality of catheter segments. In order to allow the distal and proximal ends of the catheter to have different flexibilities, usually the outer layer and/or the inner layer are formed from connecting segments made of materials with different hardness. Moreover, the distal end of the catheter needs to be as flexible as possible, and the overall catheter needs to have a good transition of flexibility. However, due to the material and design characteristics, the axial modulus of flexible catheter is low, and the joints (e.g., joints between segments of the inner or outer layer or transitions of a certain characteristic) of the plurality of catheter segments and the thinner parts of the tubular body of the catheter tend to become weak points for stress. The weak points are prone to axial deformation or even torn off under the action of axial force during clinical operation. Therefore, in an embodiment of the present disclosure, a plurality of axial components 120 may preferably be arranged symmetrically or asymmetrically along the circumference of the spring component 110 of the catheter at predetermined positions, and/or the axial components 120 are sequentially arranged at intervals along the axial direction of the spring component 110 at predetermined positions. According to the invention, the predetermined positions are at the joints of the plurality of the catheter segments in the catheter reinforcement layer (e.g., joints between segments of the inner or outer layer or transitions of a certain characteristic), the positions where the inner layer or the outer layer has a higher flexibility, the positions where the thickness of the inner or outer layer is smaller, the positions where the modulus value of the catheter is lower, and the positions where the tubular body of the catheter is thinner. Please see the following embodiments for details.

### Embodiment 4

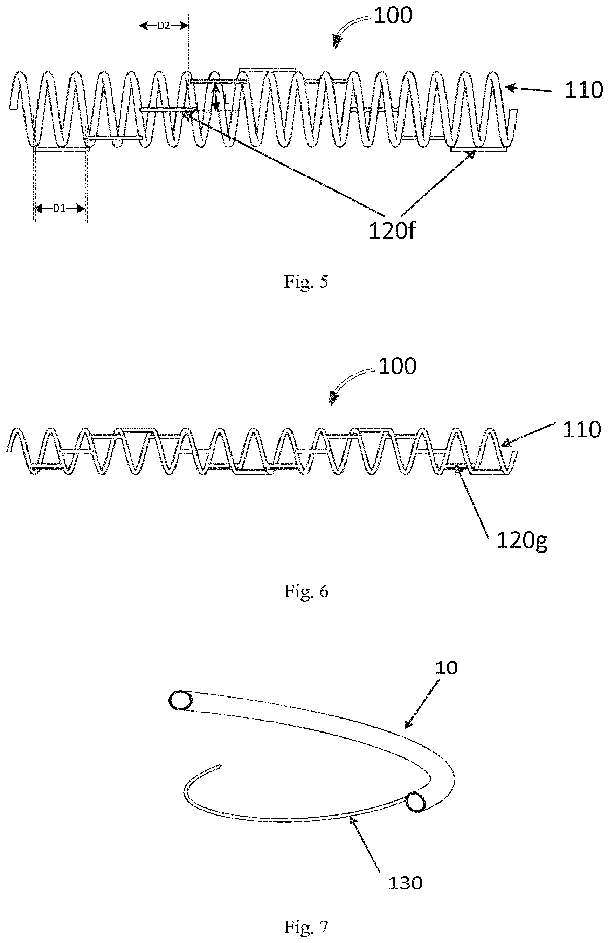

Referring to Figs. 5 and 6, which are structural schematic diagrams of a catheter reinforcement layer according to another embodiment of the present disclosure. As shown in Fig. 5 or Fig. 6, when a plurality of axial components 120 are included, the plurality of axial components 120g or axial components 120f may be arranged spirally along the circumferential direction of the spring component 110, and the plurality of axial components 120g or axial components 120f may be located between the circumferential surface or the clearance of the spring component 110 where the joints of the inner or outer layer of the adjacent catheter segments are located, and the plurality of axial components 120g or axial components 120f, except for the ends thereof, may be connected to the circumferential surface the spring component 110 at the joints thereof. Optionally, the plurality of axial components 120g or axial components 120f may be located between the circumferential surface or the clearance of the spring component 110 where the modulus value of the catheter segment is lower than that of the adjacent catheter segment at one or both sides, and between the circumferential surface or the clearance of the spring component 110 where the diameter of the catheter segment is lower than that of the adjacent catheter segment at one or both sides. Optionally, the plurality of axial components 120g or axial components 120f may be located at a part of the inner or outer layer of the catheter with a smaller thickness, or between the circumferential surface or the clearance of the spring component where the catheter segment is of a greater flexibility.

Specifically, each axial component 120g or axial component 120f may be arranged between the spring component 110 and the inner layer of the catheter, or between the spring component 110 and the outer layer 200 of the catheter, or between the clearance of the spring component 110. In addition, the axial length of each axial component 120 may be equal to the axial distance between two turns of the spring component 110 which is turned spirally in a clockwise manner, referring to the axial component 120g shown in Fig. 6. Optionally, the axial length of each axial component 120 may be equal to the axial distance between two peaks of the spring component 110 in the form of a sinusoidal wave, referring to the axial component 120f shown in Fig. 5.

It can be understood that, when a plurality of axial components are included, the axial distance between the plurality of axial components 120 may be the same or different, and/or the circumferential angular distances of the plurality of axial components 120 may be the same or different. Moreover, in the plurality of axial components 120, the axial distance of the axial components 120 located at the proximal end may be smaller than that of the axial components located at the distal end, and/or the circumferential angular distance of the axial components at the proximal end may be smaller than the axial distance of the axial components at the distal end. When a plurality of the axial components are included, the axial distance between two adjacent axial components 120g (or axial components 120f) in the axial direction of the spring component 110 is in a range of 0.00254 - 0.254 cm (0.001-0.1 inches).

It should be emphasized that the axial distance mentioned in this specification refers to the distance between the proximal ends of two axial components in a direction parallel to the axial direction, see D1 and D2 in Fig. 5, and the circumferential angular distance of two adjacent axial components in the axial direction thereof refers to the radian corresponding to L (that is, the central angle formed from two points of two axial components on a same circumference).

Optionally, the axial component 120 may be a monofilament or a twisted filament composed of multiple monofilaments. The diameter of the monofilament is in a range of 0.00127 - 0.00762 cm (0.0005-0.003 inches), and the number of monofilaments included in the twisted filament may be in a range of 1~20.

Exemplarily, the researchers of the present invention obtain the following table through experiments to show the influence of the axial component on various parameters of the catheter in the prior art. In this experiment, the specific arrangement of the axial component is shown in Fig. 3a, the axial component extends from the proximal end to the distal end along the axial direction of the braided component 110 and extends across the spring component 110. When the number of axial components is multiple (for example, N=2 or 3), the multiple axial components are arranged in parallel to each other.

**Table 1**

| Number of Axial Component | Breaking Force | Outer Diameter | Flexibility |
|---|---|---|---|
| N=1 | +0.6% | +0.1% | -0.6% |
| N=2 | +2.4% | +0.2% | -1.1% |
| N=3 | +5.6% | +0.4% | -1.6% |
| N=10 | +49.9% | +0.41% | -6.43% |
| N=15 | +57.3% | +0.46% | -9.07% |

It can be seen from the above Table 1 that, compared with the catheter without axial components, with the increase of the number of axial components, the axial breaking force of the catheter gradually increases, and the outer diameter and softness of the catheter remain basically unchanged. Therefore, adding axial components in the catheter can effectively increase the axial breaking force of the catheter with negligible influence on the outer diameter and softness of the catheter.

It can be understood that the axial components 120 shown in the figures can be connected to the spring component 100 by means of gluing, polymerization, bonding or laser welding, or can be integrally formed with the spring component 110 by cutting for example a tube.

In addition, please refer to Fig. 7, which is a structural schematic view of a catheter according to an embodiment of the present invention. As shown in Fig. 7, in any one of the above-mentioned embodiments, the axial component 120 may also include a control wire 130 extended reversely from the side close to the catheter operator (that is, the control wire 130 extends from the end of the spring component close to the operator, and the extension direction is opposite to the spiral direction of the spring component), so that the catheter operator can dynamically adjust the rotation direction of the catheter 10 through the control wire 130, so that the catheter is easier to pass tortuous vascular area. The length of the control wire 130 can be specifically determined according to actual needs, which is not specifically limited in the present invention.

In summary, the catheter reinforcement layer and the catheter according to the present invention have a novel reinforcement layer, which increases the axial modulus of the catheter by introducing one or more axial components into the reinforcement layer of the spring component of the existing catheter. It avoids the axial deformation or even breakage of the catheter caused from the axial force of the tubular body of the catheter. In addition, one or more axial components may be arranged on the mechanically weak point of the catheter, it is thus possible to prevent stress concentration on the catheter, thereby preventing the breakage of the catheter during the delivery or withdrawal of a device.

Moreover, introducing one or more axial components extending in the axial direction into the catheter reinforcement layer can improve the efficiency in transmission of the axial force of the catheter and optimize the transmission performance of the catheter. When the thickness of the inner layer of the catheter is particularly small, one or more axial components extending in the axial direction are introduced into the reinforcement layer of the catheter, which can not only ensure the flexibility of the catheter, but also prevent the axial deformation and breakage of the catheter.

It should be noted that although the present invention has been disclosed above with preferred embodiments, the above embodiments are not intended to limit the present invention. The scope of the invention is defined by the appended claims.

It should also be understood that, unless otherwise specified or pointed out, the terms "first", "second", "third" and other descriptions in the specification are only used to distinguish each component, element, step, etc. in the specification, not used to express the logical relationship or sequence relationship between various components, elements, and steps.

In addition, it should be understood that the terminology described herein is used to describe particular embodiments only and is not intended to limit the scope of the invention. It must be noted that as used herein and in the appended claims, the singular forms "a" and "an" include plural referents unless the context clearly dictates otherwise. For example, a reference to "a step" or "a means" means a reference to one or more steps or means, and may include sub-steps as well as sub-means. All conjunctions used should be understood in their broadest sense. And, the word "or" should be understood as having the definition of logical "or" rather than logical "exclusive or", unless the context clearly expresses the contrary meaning. ,

## Claims

1. A catheter (10), comprising an inner layer, a reinforcement layer and an outer layer (200) that are sequentially arranged from inside to outside and are all in a shape of a tube, and wherein the reinforcement layer comprises a catheter reinforcement layer (100), the catheter reinforcement layer (100) comprising a spring component (110) and a plurality of axial components (120), wherein each of the plurality of axial components (120) extends along the spring component (110) from a proximal end to a distal end, and has at least one intersection with the spring component (110), wherein
the catheter (10) comprises a plurality of catheter segments connected in series,
**characterized in that**
the axial components (120) are arranged at predetermined positions, the predetermined positions are at joints of the plurality of the catheter segments, positions where the inner layer or the outer layer has a higher flexibility, positions where a thickness of the inner layer or the outer layer is smaller, position where a modulus value of the catheter (10) is lower, and positions where the tube of the catheter (10) is thinner.

2. The catheter (10) according to claim 1, wherein a material of the plurality of axial components (120) is metal and/or polymer filament; or
wherein each of the plurality of axial components (120) has a filamentous structure shaped as any one or a combination of a straight line, wave line or spiral line; or
wherein the axial member has 1-80,000 said intersections with the spring component (110).

3. The catheter (10) according to claim 1, wherein each of the plurality of axial components (120) is arranged parallel to an axial direction of the spring component (110); or
each of the plurality of axial components (120) is arranged at an angle of 0-45° with an axial direction of the spring component (110); or
at least a part of the plurality of axial components (120) is arranged at an angle of 0-45° with an axial direction of the spring component (110), and other parts of the plurality of axial components (120) are parallel to the axial direction of the spring component (110).

4. The catheter (10) according to claim 1, wherein each of the plurality of axial components (120), from the proximal end to the distal end, is shaped as a straight line or spiral line attached to an inner surface or an outer surface of the spring component (110); or
each of the plurality of axial components (120), from the proximal end to the distal end, is shaped as a wave line attached between an inner surface and an outer surface of the spring component (110) in a staggered manner; or
each of the plurality of axial components (120), from the proximal end to the distal end, is arranged in a clearance of the spring component (110), and the plurality of axial components (120) and the spring component (110) are in a same plane.

5. The catheter (10) according to claim 1, wherein
the plurality of axial components (120) are arranged symmetrically or asymmetrically along a circumference of the spring component (110); and/or
the plurality of axial components (120) are sequentially arranged at intervals along an axial direction of the spring component (110).

6. The catheter (10) according to claim 1, wherein
the plurality of axial components (120) are arranged symmetrically or asymmetrically along a circumference of the spring component (110) at the predetermined positions; and/or
the plurality of axial components (120) are sequentially arranged at intervals along an axial direction of the spring component (110) at the predetermined positions.

7. The catheter (10) according to claim 1, wherein the plurality of axial components (120) are arranged spirally along an axial direction of the spring component (110).

8. The catheter (10) according to claim 7, wherein axial distances between the plurality of axial components (120) are the same or different, and/or
circumferential angular distances between the plurality of axial components (120) are the same or different.

9. The catheter (10) according to claim 8, wherein the axial distances between the axial components (120) located at the proximal end is smaller than the axial distances of the axial components (120) located at the distal end; and/or
the circumferential angular distances between the axial components (120) located at the proximal end is smaller than the axial distances of the axial components (120) located at the distal end.

10. The catheter (10) according to claim 1, wherein a number of the axial components (120) ranges from 2 to 16,000; or
wherein an axial distance between two axial components (120) adjacent in an axial direction of the spring component (110) is in a range of 0.00254 - 0.254 cm (0.001-0.1 inches); or
wherein a material of at least one of the at least one axial member is a radioautographic material.

11. The catheter (10) according to any one of claims 1-10, wherein each of the plurality of axial components (120) is a monofilament or a twisted filament composed of a plurality of monofilaments.

12. The catheter (10) according to claim 11, wherein a diameter of the monofilament is in a range of 0.00127 - 0.00762 cm (0.0005-0.003 inches), and a number of the monofilaments contained in the twisted filament is in a range of 1-20.

13. The catheter (10) according to claim 1, wherein each of the plurality of axial component (120) is connected to the spring component (110) by means of gluing, polymerization, bonding or laser welding, or is integrally formed with the spring component (110) by means of cutting.

14. The catheter (10) according to claim 1, wherein the plurality of axial components (120) comprises a control wire (130) extended reversely from a side close to a catheter operator, so that the catheter operator can adjust a rotation direction of the catheter (10) through the control wire (130).

15. The catheter (10) according to claim 1, wherein the inner layer is a polymer material layer having a thickness of 0.000254 - 0.00508 cm (0.0001-0.002 inches), preferably 0.000762 - 0.001524 cm (0.0003-0.0006 inches).

## Patentansprüche

1. Katheter (10), der eine innere Schicht, eine Verstärkungsschicht und eine äußere Schicht (200) umfasst, die von innen nach außen nacheinander angeordnet sind und alle die Form einer Röhre haben, und wobei die Verstärkungsschicht eine Katheterverstärkungsschicht (100) umfasst, die Katheterverstärkungsschicht (100) eine Federkomponente (110) und eine Vielzahl von axialen Komponenten (120) umfasst, wobei sich jede der Vielzahl von axialen Komponenten (120) entlang der Federkomponente (110) von einem proximalen Ende zu einem distalen Ende erstreckt und mindestens eine Schnittstelle mit der Federkomponente (110) aufweist, wobei
der Katheter (10) eine Vielzahl von in Reihe geschalteten Kathetersegmenten umfasst,
**dadurch gekennzeichnet, dass**
die axialen Komponenten (120) an vorbestimmten Positionen angeordnet sind, wobei die vorbestimmten Positionen an Verbindungsstellen der Vielzahl von Kathetersegmenten liegen, Positionen, wo die innere Schicht oder die äußere Schicht eine höhere Flexibilität aufweist, Positionen, wo eine Dicke der inneren Schicht oder der äußeren Schicht geringer ist, Positionen, wo ein Modulwert des Katheters (10) niedriger ist, und Positionen, wo der Schlauch des Katheters (10) dünner ist.

2. Katheter (10) nach Anspruch 1, wobei ein Material der Vielzahl von axialen Komponenten (120) Metall und/oder Polymerfilament ist; oder
wobei jede von der Vielzahl von axialen Komponenten (120) eine filamentartige Struktur aufweist, die wie eines von einer geraden Linie, einer Wellenlinie oder einer Spirallinie oder einer Kombination davon geformt ist; oder
wobei das axiale Element 1-80 000 der Schnittstellen mit der Federkomponente (110) aufweist.

3. Katheter (10) nach Anspruch 1, wobei jede von der Vielzahl von axialen Komponenten (120) parallel zu einer Axialrichtung der Federkomponente (110) angeordnet ist; oder
jede von der Vielzahl von axialen Komponenten (120) in einem Winkel von 0-45° zu einer Axialrichtung der Federkomponente (110) angeordnet ist; oder
mindestens ein Teil der Vielzahl von axialen Komponenten (120) in einem Winkel von 0-45° zu einer Axialrichtung der Federkomponente (110) angeordnet ist und andere Teile der Vielzahl von axialen Komponenten (120) parallel zur Axialrichtung der Federkomponente (110) sind.

4. Katheter (10) nach Anspruch 1, wobei jede von der Vielzahl von axialen Komponenten (120) vom proximalen Ende bis zum distalen Ende als gerade Linie oder Spirallinie geformt ist, die an einer Innenfläche oder einer Außenfläche der Federkomponente (110) befestigt ist; oder
jede von der Vielzahl von axialen Komponenten (120) vom proximalen Ende bis zum distalen Ende als eine Wellenlinie geformt ist, die zwischen einer Innenfläche und einer Außenfläche der Federkomponente (110) in versetzter Form angebracht ist; oder
jede der mehreren axialen Komponenten (120) vom proximalen Ende bis zum distalen Ende in einem Zwischenraum der Federkomponente (110) angeordnet ist und die Vielzahl von axialen Komponenten (120) und die Federkomponente (110) in derselben Ebene liegen.

5. Katheter (10) nach Anspruch 1, wobei
die Vielzahl von axialen Komponenten (120) symmetrisch oder asymmetrisch entlang eines Umfangs der Federkomponente (110) angeordnet ist; und/oder
die Vielzahl von axialen Komponenten (120) in Abständen entlang einer Axialrichtung der Federkomponente (110) nacheinander angeordnet sind.

6. Katheter (10) nach Anspruch 1, wobei
die Vielzahl von axialen Komponenten (120) symmetrisch oder asymmetrisch entlang eines Umfangs der Federkomponente (110) an den vorbestimmten Positionen angeordnet ist; und/oder
die Vielzahl von axialen Komponenten (120) in Abständen entlang einer Axialrichtung der Federkomponente (110) an den vorbestimmten Positionen nacheinander angeordnet ist.

7. Katheter (10) nach Anspruch 1, wobei die Vielzahl von axialen Komponenten (120) spiralförmig entlang einer Axialrichtung der Federkomponente (110) angeordnet ist.

8. Katheter (10) nach Anspruch 7, wobei die axialen Abstände zwischen der Vielzahl von axialen Komponenten (120) gleich oder unterschiedlich sind, und/oder
Umfangswinkelabstände zwischen der Vielzahl von axialen Komponenten (120) gleich oder unterschiedlich sind.

9. Katheter (10) nach Anspruch 8, wobei die axialen Abstände zwischen den am proximalen Ende befindlichen axialen Komponenten (120) kleiner sind als die axialen Abstände der am distalen Ende befindlichen axialen Komponenten (120); und/oder
die Umfangswinkelabstände zwischen den am proximalen Ende befindlichen axialen Komponenten (120) kleiner sind als die axialen Abstände der am distalen Ende befindlichen axialen Komponenten (120).

10. Katheter (10) nach Anspruch 1, wobei eine Anzahl der axialen Komponenten (120) von 2 bis 16 000 reicht; oder
wobei ein axialer Abstand zwischen zwei axialen Komponenten (120), die in einer Axialrichtung der Federkomponente (110) benachbart sind, in einem Bereich von 0,00254-0,254 cm (0,001-0,1 Zoll) liegt; oder
wobei ein Material mindestens eines von dem mindestens einen axialen Element ein radioautographisches Material ist.

11. Katheter (10) nach einem der Ansprüche 1 bis 10, wobei jede von der Vielzahl von axialen Komponenten (120) ein Monofilament oder ein aus einer Vielzahl von Monofilamenten zusammengesetztes verdrilltes Filament ist.

12. Katheter (10) nach Anspruch 11, wobei ein Durchmesser des Monofilaments in einem Bereich von 0,00127-0,00762 cm (0,0005-0,003 Zoll) liegt und eine Anzahl der im verdrillten Filament enthaltenen Monofilamente in einem Bereich von 1-20 liegt.

13. Der Katheter (10) nach Anspruch 1, wobei jede von der Vielzahl von axialen Komponenten (120) durch Kleben, Polymerisieren, Verbinden oder Laserschweißen mit der Federkomponente (110) verbunden ist oder durch Schneiden integral mit der Federkomponente (110) geformt ist.

14. Katheter (10) nach Anspruch 1, wobei die Vielzahl von axialen Komponenten (120) einen Steuerdraht (130) umfasst, der sich von einer Seite in der Nähe eines Katheterbedieners nach hinten erstreckt, so dass der Katheterbediener eine Drehrichtung des Katheters (10) durch den Steuerdraht (130) einstellen kann.

15. Katheter (10) nach Anspruch 1, wobei die innere Schicht eine Polymermaterialschicht ist, die eine Dicke von 0,000254-0,00508 cm (0,0001-0,002 Zoll), vorzugsweise 0,000762-0,001524 cm (0,0003-0,0006 Zoll) aufweist.

## Revendications

1. Cathéter (10), comprenant une couche interne, une couche de renfort et une couche externe (200) qui sont disposées séquentiellement de l'intérieur vers l'extérieur et sont toutes en forme de tube, et dans lequel la couche de renfort comprend une couche de renfort de cathéter (100), la couche de renfort de cathéter (100) comprenant un composant de ressort (110) et une pluralité de composants axiaux (120), dans lequel chacun de la pluralité de composants axiaux (120) s'étend le long du composant de ressort (110) d'une extrémité proximale à une extrémité distale, et présente au moins une intersection avec le composant de ressort (110), dans lequel
le cathéter (10) comprend une pluralité de segments de cathéter reliés en série,
**caractérisé en ce que**
les composants axiaux (120) sont disposés dans des positions prédéterminées, les positions prédéterminées sont, au niveau des articulations de la pluralité des segments de cathéter, des positions où la couche interne ou la couche externe présente une flexibilité plus élevée, des positions où une épaisseur de la couche interne ou de la couche externe est plus petite, une position où une valeur de module du cathéter (10) est plus faible, et des positions où le tube du cathéter (10) est plus mince.

2. Cathéter (10) selon la revendication 1, dans lequel un matériau de la pluralité de composants axiaux (120) est du métal et/ou un filament polymère ; ou
dans lequel chacun de la pluralité de composants axiaux (120) présente une structure filamenteuse formée comme une ligne droite, une ligne ondulée ou une ligne en spirale ou une combinaison de celles-ci ; ou
dans lequel l'élément axial présente 1-80 000 dites intersections avec le composant de ressort (110).

3. Cathéter (10) selon la revendication 1, dans lequel chacun de la pluralité de composants axiaux (120) est disposé parallèlement à une direction axiale du composant de ressort (110) ; ou
chacun de la pluralité de composants axiaux (120) est disposé selon un angle de 0-45° avec une direction axiale du composant de ressort (110) ; ou
au moins une partie de la pluralité de composants axiaux (120) est disposée selon un angle de 0-45° avec une direction axiale du composant de ressort (110), et d'autres parties de la pluralité de composants axiaux (120) sont parallèles à la direction axiale du composant de ressort (110).

4. Cathéter (10) selon la revendication 1, dans lequel chacun de la pluralité de composants axiaux (120), de l'extrémité proximale à l'extrémité distale, est formé comme une ligne droite ou une ligne en spirale fixée à une surface intérieure ou extérieure du composant de ressort (110) ; ou
chacun de la pluralité de composants axiaux (120), de l'extrémité proximale à l'extrémité distale, est formé comme une ligne d'onde fixée entre une surface intérieure et une surface extérieure du composant de ressort (110) de manière décalée ; ou
chacun de la pluralité de composants axiaux (120), de l'extrémité proximale à l'extrémité distale, est disposé dans un jeu du composant de ressort (110), et la pluralité de composants axiaux (120) et le composant de ressort (110) sont dans un même plan.

5. Cathéter (10) selon la revendication 1, dans lequel
la pluralité de composants axiaux (120) sont disposés symétriquement ou asymétriquement le long d'une circonférence du composant de ressort (110) ; et/ou
la pluralité de composants axiaux (120) sont disposés séquentiellement à intervalles le long d'une direction axiale du composant de ressort (110).

6. Cathéter (10) selon la revendication 1, dans lequel
la pluralité de composants axiaux (120) sont disposés symétriquement ou asymétriquement le long d'une circonférence du composant de ressort (110) dans des positions prédéterminées ; et/ou
la pluralité de composants axiaux (120) sont disposés séquentiellement à intervalles le long d'une direction axiale du composant de ressort (110) dans des positions prédéterminées.

7. Cathéter (10) selon la revendication 1, dans lequel la pluralité de composants axiaux (120) sont disposés en spirale le long d'une direction axiale du composant de ressort (110).

8. Cathéter (10) selon la revendication 7, dans lequel les distances axiales entre la pluralité de composants axiaux (120) sont identiques ou différentes, et/ou
les distances angulaires circonférentielles entre la pluralité de composants axiaux (120) sont identiques ou différentes.

9. Cathéter (10) selon la revendication 8, dans lequel les distances axiales entre les composants axiaux (120) situés à l'extrémité proximale sont inférieures aux distances axiales des composants axiaux (120) situés à l'extrémité distale ; et/ou
les distances angulaires circonférentielles entre les composants axiaux (120) situés à l'extrémité proximale sont inférieures aux distances axiales des composants axiaux (120) situés à l'extrémité distale.

10. Cathéter (10) selon la revendication 1, dans lequel le nombre de composants axiaux (120) est compris entre 2 et 16 000 ; ou
dans lequel une distance axiale entre deux composants axiaux (120) adjacents dans une direction axiale du composant de ressort (110) est dans une plage de 0,00254-0,254 cm (0,001-0,1 pouce) ; ou
dans lequel un matériau d'au moins un de l'au moins un élément axial est un matériau radioautographique.

11. Cathéter (10) selon l'une quelconque des revendications 1-10, dans lequel chacun de la pluralité de composants axiaux (120) est un monofilament ou un filament torsadé composé d'une pluralité de monofilaments.

12. Cathéter (10) selon la revendication 11, dans lequel un diamètre du monofilament est dans une plage de 0,00127-0,00762 cm (0,0005-0,003 pouce), et un nombre de monofilaments contenus dans le filament torsadé est dans une plage de 1-20.

13. Cathéter (10) selon la revendication 1, dans lequel chacun des composants axiaux (120) est relié au composant de ressort (110) par collage, polymérisation, liaison ou soudage au laser, ou est formé d'un seul tenant avec le composant de ressort (110) par découpe.

14. Cathéter (10) selon la revendication 1, dans lequel la pluralité de composants axiaux (120) comprennent un fil de commande (130) s'étendant en sens inverse depuis un côté proche d'un opérateur de cathéter de sorte que l'opérateur de cathéter puisse ajuster une direction de rotation du cathéter (10) par l'intermédiaire du fil de commande (130).

15. Cathéter (10) selon la revendication 1, dans lequel la couche interne est une couche de matériau polymère présentant une épaisseur de 0,000254-0,00508 cm (0,0001-0,002 pouce), de préférence de 0,000762-0,001524 cm (0,0003-0,0006 pouce).
